## Europäisches Patentamt
## European Patent Office
## Office européen des brevets

(19)

(11) Veröffentlichungsnummer: **0 133 982**
**B1**

(12) **EUROPÄISCHE PATENTSCHRIFT**

(45) Veröffentlichungstag der Patentschrift:
20.05.87

(51) Int. Cl.⁴: **C 07 C 11/24**, C 10 J 3/46,
C 10 J 3/18

(21) Anmeldenummer: **84109029.3**

(22) Anmeldetag: **31.07.84**

(54) **Verfahren zur Erzeugung von Acetylen und Synthese- oder Reduktionsgas aus Kohle in einem Lichtbogenprozess.**

(30) Priorität: **26.08.83 DE 3330750**

(43) Veröffentlichungstag der Anmeldung:
**13.03.85 Patentblatt 85/11**

(45) Bekanntmachung des Hinweises auf die Patenterteilung:
**20.05.87 Patentblatt 87/21**

(84) Benannte Vertragsstaaten:
**AT BE DE FR GB IT NL SE**

(56) Entgegenhaltungen:
**EP-A-0 031 588**
**FR-A-2 177 089**
**FR-A-2 376 200**
**GB-A-2 101 151**

**ERDÖL UND KOHLE ERDGAS-PETROCHEMIE,
Band 34, Nr. 6, Seiten 237-242, Leinfelden-
Echterdingen; D. BITTNER et al.:
"Direktumwandlung von Kohle zu Acetylen"**

(73) Patentinhaber: **HÜLS AKTIENGESELLSCHAFT, -
RSP Patente / PB 15 - Postfach 13 20, D-4370
Marl 1 (DE)**

(72) Erfinder: **Müller, Richard, Dr., Im Singelsen 24,
D-4370 Marl (DE)**
Erfinder: **Kerker, Lothar, Dr., Dechant- Wieling-
Strasse 9, D-4408 Dülmen (DE)**
Erfinder: **Peuckert, Cornelius, Dr., Oststrasse 48,
D-4220 Dinslaken (DE)**

## Beschreibung

Angesichts der zukünftig zu erwartenden Verknappung an fossilen Rohstoffen gewinnen Verfahren an Interesse, die das Einkoppeln elektrischer Energie, die auch auf nicht fossiler Basis, wie Kernkraft oder regenerativen Quellen, wie Wasserkraft und Solarenergie gewonnen werden kann, in energieverbrauchende Prozesse ermöglichen. Wird diese Energie bei hohen Temperaturen benötigt, so sind Lichtbogen- oder Plasmaprozesse besonders geeignet. So ist die Erzeugung von Acetylen aus gasförmigen und flüssigen Kohlenwasserstoffen schon seit Jahren bekannt und wird großtechnisch betrieben (Gladisch, Hydrocarbon Processing, Petroleum Refiner 41, Nr. 6, 159 bis 164 (1962)). Bei diesem Verfahren kann etwa 50 % des Bedarfs an fossilen Rohstoffen gegenüber vergleichbaren Prozessen, die ganz auf fossiler Basis beruhen, wie z. B. der partiellen Oxidation, eingespart werden. In den letzten Jahren sind Entwicklungsarbeiten begonnen worden, um auch aus Kohle in einem Lichtbogen- oder Plasmaverfahren Acetylen herzustellen (D. Bittner, H. Baumann, C. Peuckert, J. Klein, H. Jüntgen, Erdöl und Kohle Erdgas-Petrochemie 34, Heft 6, 237 bis 242 (1981)).

Eine weitere Reaktion, bei der Plasmaverfahren Anwendung gefunden haben, ist das Reformieren von Kohlenwasserstoffen oder Kohle mittels eines Vergasungsmittels, wie Wasserdampf oder Kohlendioxid zu einem Gasgemisch, das überwiegend aus CO und $H_2$ besteht und in der chem. Industrie als Synthesegas bzw. in der metallurgischen Industrie als Reduktionsgas breite technische anwendung findet.

Auch hier kann durch Einsatz des Lichtbogenverfahrens ca. 50 % des direkten Bedarfs an fossiler Energie eingespart werden.

Bei der Pyrolyse von Kohle im Plasmaverfahren können wesentlich größere Mengen an flüchtigen Kohlenwasserstoffen aus der Kohle gewonnen werden, als bei den üblichen Verkokungsprozessen. Ein Maß für den Anteil der üblicherweise gewinnbaren Verbindungen ist der unter genormten Bestimmungsverfahren ermittelte Gehalt an sogenannten "Flüchtigen" der Kohle. Bei der Plasmapyrolyse läßt sich eine bis etwa zum Faktor 2 höhere Ausbeute an flüchtigen Verbindungen gewinnen, als die "Flüchtigen" der Kohle ausweisen. Hierbei bestehen diese Verbindungen überwiegend aus $C_2H_2$ und CO. Als Rückstand bleibt Koks zurück. Dennoch fallen damit bei der technischen Durchführung noch ca. 1 bis 2 t Koks/t $C_2H_2$ an, der entsorgt werden muß. Wird dieser Koks in Kraftwerken verbrannt, so wird dadurch bis zu 50 % des elektrischen Energiebedarfs für die Acetylenerzeugung, der bei ca. 10 kWh/kg $C_2H_2$ liegt, gewonnen. Damit geht aber ein großer Teil des Vorteils eines Lichtbogenverfahrens, nämlich die Einkopplung elektrischer Energie auf nicht fossiler Basis, wieder verloren.

Es besteht also die Aufgabe, für den in einer Anlage zur Acetylenerzeugung aus Kohle im Lichtbogen- bzw. Plasmaverfahren anfallenden Koks eine Verwendungsmöglichkeit zu suchen, die sich gut in eine chem. Fabrik eingliedern läßt, und die eine optimale Einbindung insbesondere von auf nicht fossiler Basis erzeugter elektrischer Energie ermöglicht.

Diese Aufgabe wird erfindungsgemäß entsprechend den Angaben der Patentansprüche gelöst.

Zu feinkörnigem Pulver, wie beispielsweise für Kohlestaubbrenner üblich (90 % < 100 μ), aufbereitete Kohle wird zunächst in einer ersten Stufe in einem Lichtbogen- oder Plasmaverfahren bei einer Verweilzeit von 0,5 bis 10 msec, vorzugsweise von 1,0 bis 2 msec und Temperaturen von mindestens 1 500 °C, vorzugsweise von 1 500 bis 3 000 °C zur Acetylenerzeugung eingesetzt. Der hierbei anfallende Koks wird abgetrennt und dann in einer zweiten Stufe unter Anwendung eines Lichtbogenverfahrens mit einem Vergasungsmittel bei einer Temperatur von mindestens 800 °C, vorzugsweise von 800 bis 1 700 °C und einer Verweilzeit von 1 bis 15 sec, vorzugsweise von 2 bis 6 sec, zu einem Reduktions- oder Synthesegas umgesetzt. Gegen ein solches Verfahren spricht jedoch die bekannte Tatsache, daß der sogar bei tieferen Temperaturen hergestellte Koks eine wesentlich geringere Vergasungsgeschwindigkeit hat als die Kohle, was auf den geringen Anteil an Flüchtigen, aber auch auf die "Sinterung" des Kohlenstoffgerüstes bei der Verkokungstemperatur von 900 bis 1 200 °C zurückzuführen ist.

So beschreiben z. B. H. Herlitz und S. Santen (Plasmatechnology for production of synthesisgas from coal and other fuels, Seminar on Chemicals from Synthesisgas, ECI, 14.06.83) ein Lichtbogenverfahren zur Kohlevergasung, bei dem der Kohle in einem Schachtofen Koks zugesetzt wird, wobei der Koksumsatz nur 7 bis 10 % des Kohleumsatzes beträgt.

Da bei der Acetylenherstellung aus Kohle im Lichtbogenverfahren die Reaktion bei wesentlich höheren Temperaturen, über 1 500 °C, durchgeführt wird, bestand wenig Aussicht, daß der bei der Plasmapyrolyse anfallende Koks in technisch vertretbaren Verweilzeiten, die im Bereich von Sekunden liegen, mit einem Lichtbogenverfahren vergast werden kann.

Es wurde nun überraschenderweise gefunden, daß sich dieser Nachteil vermeiden läßt, wenn man die Pyrolysereaktion in einer ersten Stufe bei einer Energiedichte von 1 bis 5 kWh/Nm³, einer Temperatur von mindestens 1 500 °C und einer Verweilzeit von 0,5 bis 10 msec so durchführt, daß die Ausbeute an gasförmigen Verbindungen das 1,8fache der sogenannten "Flüchtigen" Bestandteile der Kohle, vorzugsweise das 1,1- bis 1,8fache, nicht übersteigt.

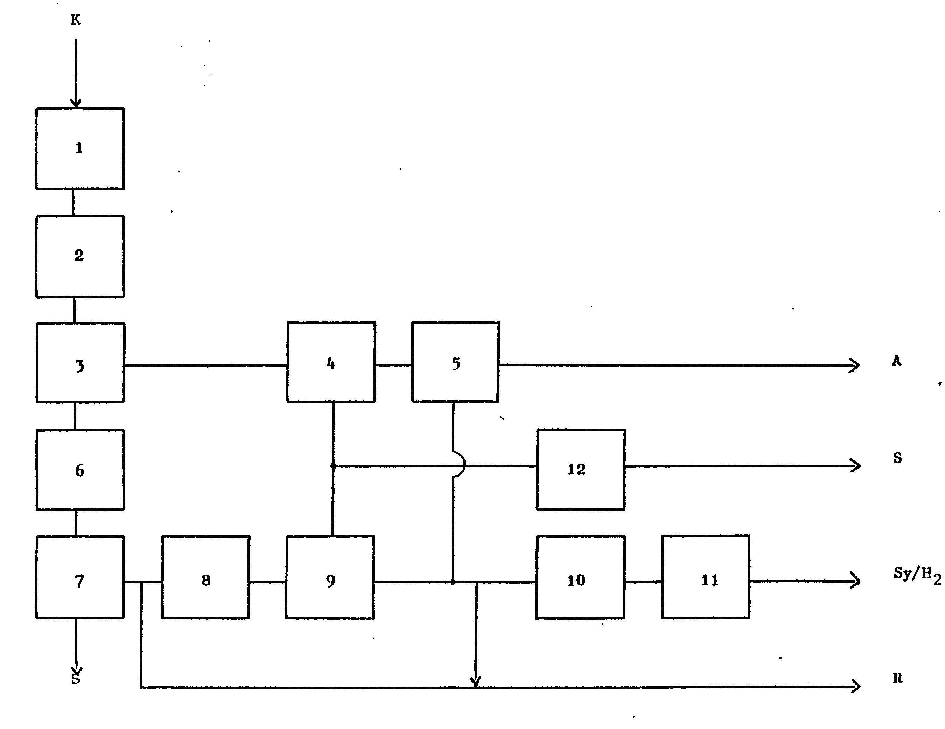

Das Verfahren wird anhand der Abb. 1 beschrieben.

Gemahlene, pulverförmige und getrocknete Kohle (Korngröße 90 % < 100 µ;Ⓡ) wird mittels eines Fördergases, das höchstens Spuren an oxidierenden Bestandteilen enthält, beispielsweise Wasserstoff, CO, $CH_4$ oder andere gasförmige Kohlenwasserstoffe in einen Lichtbogenreaktor ① eingedüst, der sowohl in ein- als auch zweistufiger Form betrieben werden kann.

Beim einstufigen Reaktor wird die Kohle mit dem Fördergas vom Lichtbogen direkt aufgeheizt, wohingegen beim zweistufigen Reaktor die Energie zunächst an ein Plasmagas übertragen wird und in der zweiten Stufe die Kohle mit dem Fördergas in den heißen Plasmastrahl eingedüst wird.

Als Einsatz ist prinzipiell jede Kohle geeignet.

Als Plasmagas sind $H_2$, CO, Kohlenwasserstoffe, wie $CH_4$ oder andere gesättigte und ungesättigte Kohlenwasserstoffe und $N_2$ sowie deren Gemische geeignet.

Die Reaktionsbedingungen im Lichtbogenreaktor werden so gewählt, daß die Ausbeute an gasförmigen Verbindungen nicht mehr als das 1,8fache der flüchtigen Bestandteile beträgt. Die hierzu notwendigen Bedingungen können auf vielfältige Weise eingestellt werden, die dem Fachmann geläufig sind. So kann z. B. nach der Dissertation von C. Peuckert (Aachen 1980) die Energiedichte des Plasmastrahls auf 1 bis 5 kWh/Nm³, vorzugsweise 2,0 bis 3,5 kWh/Nm³ und damit dessen Temperatur auf mindestens 1 500 °C, vorzugsweise 1 500 bis 3 000 °C erhöht werden, um das Ausbringen an flüchtigen Bestandteilen aus der Kohle zu erhöhen. Andere dem Fachmann naheliegende Maßnahmen sind die Erhöhung des Energieangebotes, bezogen auf die eingesetzte Kohle, beispielsweise von 1,0 auf 5 kWh/kg Kohle, die Erhöhung der Verweilzeit von 0,1 auf 10 msec, vorzugsweise 0,5 bis 5 msec, oder die Druckabsenkung von 1,3 bis 0,1 bar, wobei man bei einer längeren Verweilzeit eine niedrigere Energiedichte und Temperatur bevorzugt.

Nach der Reaktionszone wird die Reaktion durch ein direktes oder indirektes Quenchenverfahren, ② wie z. B. Wasser, Flüssiggas oder einem Wärmeaustauscher wie einem Abhitzekessel mit Dampfgewinnung oder eine entsprechende Kombination unterbrochen.

Nach dieser Temperaturabsenkung, beispielsweise auf 150 bis 300 °C, wird der Koks abgetrennt ③ und das Spaltgas, das u. a. $C_2H_2$, $H_2$, CO und flüchtige S-Verbindungen wie $H_2S$ und $CS_2$ enthält, der weiteren Aufarbeitung zur Gewinnung von Acetylen Ⓐ ④ ⑤ z. B. nach dem Verfahren der DE-OS 31 50 340 ( = US-PS 4 367 363) zugeführt. Das Acetylen gewinnt man aus dem Gasstrom durch selektive Lösungsmittel. Geeignete Lösungsmittel sind beispielsweise Wasser, Methanol, N-Methylpyrolidon oder deren Gemische.

Der in der Pyrolysezone erzeugte und in der ersten Gasreinigungsstufe im Gemisch mit anderen Gasen anfallende Schwefelkohlenstoff wird vorzugsweise der Vergasungszone zugeführt. Der bei der Pyrolyse anfallende Schwefelwasserstoff kann gemeinsam mit dem bei der Vergasung anfallenden Schwefelwasserstoff zu Schwefel Ⓢ aufgearbeitet werden, beispielsweise in einer Clausanlage.

Der Koks wird über geeignete Förderorgane, wie z. B. Schnecken, mittels eines Fördergases und/oder Vergasungsmittels, das jetzt auch oxidierende Bestandteile, wie $H_2O$ oder $CO_2$ enthalten kann, oder nach Anmaischung mit Wasser in einen weiteren Lichtbogenreaktor ⑥ eingebracht. Als Plasmagas sind $H_2$, CO, $H_2O$ und/oder $CO_2$ geeignet. Bevorzugt setzt man als Plasmagas teilweise oder ganz ein Vergasungsmittel wie Wasserdampf, Kohlendioxid oder Gemische aus beiden ein. Vorzugsweise sollte das molare O/C-Verhältnis zwischen 1,1 und 1,5 liegen, insbesondere zwischen 1,1 und 1,2 liegen.

Dieser zweite Lichtbögenreaktor kann ebenfalls in ein- oder zweistufiger Form ausgeführt sein. Bei der zweistufigen Form, die z. B. in DE-OS 31 04 281 (= US-PS 4 362 554) beschrieben ist, kann der Plasmastrahl z. B. aus Wasserstoff, einem Kreisgas aus der Aufarbeitung, wie z. B. CO/$H_2$-Gemisch oder einem Vergasungsmittel, wie z. B. Wasserdampf bestehen.

Nach einer Verweilzeit von 1 bis 15 sec, vorzugsweise 2 bis 6 sec bei Temperaturen von mindestens 800 °C, vorzugsweise 800 bis 1 700 °C, insbesondere 1 000 bis 1 500 °C, wird die Schlacke (S) (7) abgetrennt. Je nach Verwendungszweck wird ein Teilstrom abgezweigt und der restliche Gasstrom über einen Wärmetauscher (8) abgekühlt, wie aus den verschiedenen Verfahren zur Kohlevergasung mittels Sauerstoff bekannt ist (B. Cornils, I. Hibbel, P. Ruprecht, R. Dürrfeld, J. Langhöff, Hydrocarbon Processing, Seite 152, Jan. 1981).

Das Gas wird dann in bekannter Weise von den sauren Bestandteilen, wie $CO_2$ und $H_2S$, gereinigt (9) und der weiteren Verwendung zugeführt. Als solche Verwendung sind bekannt:

Reduktionsgas (R) bei der Direktreduktion oder im Hochofen und Synthesegas (Sy), wie z. B. zur Oxosynthese, Methanolsynthese oder $NH_3$-Synthese, wobei gegebenenfalls eine Konvertierung (10) und eine Gastrennung (11) notwendig ist. Hierbei kann das aus der Acetylenreinigung (5) anfallende CO/$H_2$-Gemisch in die Aufarbeitungsstufen (10) und (11) vorteilhafterweise miteinbezogen werden und so ebenfalls einer geeigneten Verwendung zugeführt werden. Da bereits bei der Pyrolyse ein Teil der S-Verbindungen der Kohle zu $H_2S$ umgesetzt werden, können die entsprechenden Gasströme aus der Pyrolyse und der Vergasung einer gemeinsamen S-Gewinnungsanlage (12), wie z. B. einer Clausanlage, zugeführt werden.

**Vergleichsbeispiel A**

In einem Lichtbogenofen, der mit einer Leistung von 360 kW und der mit $H_2$ als Trägergas bei einem Druck von 1 bar betrieben wird, wird feingemahlene und getrocknete Kohle (90 % < 100 µ) in einer Menge von 100 kg/h - wasser- und aschefrei (waf) gerechnet - und einem nach DIN 51 720 bestimmten Gehalt an Flüchtigen von 30 % waf mittels eines $H_2$-Stromes bei einer Beladung von 10 kg/kg Gas in den aus diesem Lichtbogenofen austretenden Plasmastrahl, der eine Energiedichte von 4,0 kWh/Nm$^3$ besitzt, eingedüst und in einen zylindrischen Reaktor auf eine mittlere Temperatur von 2 600 °C, einer Verweilzeit von 13 Millisekunden aufgeheizt und pyrolisiert; das Gas-Koksgemisch wird durch Eindüsen von Wasser auf 200 °C abgekühlt, und der Koks in einem Zyklon abgetrennt und das Gas in bekannter Weise über Wasser- und Laugewäschen gereinigt.

Die im Anschluß hieran mittels einer Blende und einer Dichtemessung bestimmte Gasmenge beträgt 78,5 kg/h. Damit verbleibt nach Abzug der eingesetzten Gasmengen eine aus der Kohle erzeugte Gasmenge von 60 kg/h. Damit sind 60 % der Kohle bei der Pyrolyse zu flüchtigen Bestandteilen umgesetzt worden, was dem 2,0fachen der "Flüchtigen" entspricht. Die Acetylenausbeute beträgt 27 % und der für die Wirtschaftlichkeit des Verfahrens wichtige spez. Energiebedarf liegt bei 13,3 kWh/kg $C_2H_2$.

Der Koks wird dann in einen ausgemauerten, zylindrischen Reaktor mittels eines Gemisches aus 25 % $H_2O$ und 75 % $H_2$ in einer Menge von 60 kg/h, wasser- und aschefrei gerechnet, bei einer Temperatur von 150 °C am Kopf eingedüst. Gleichzeitig strömt hier in den Reaktor ein Plasmastrahl aus Wasserstoff mit einer Energiedichte von 3,0 kWh/Nm$^3$, der ebenfalls in einem Lichtbogenreaktor mit einer Leistung von 300 kW erzeugt wird. Weiterhin wird am Kopf des Reaktors Wasserdampf zugeführt, so daß, bezogen auf den Kohlenstoffgehalt des Kokses, ein molares O/C-Verhältnis von 1,15 eingehalten wird.

Der Reaktor ist so bemessen, daß die durchschnittliche Verweilzeit des Gases 6 sec beträgt. Die Ausgangstemperatur liegt bei 1 300 °C. Ein Teil der Schlacke sammelt sich in flüssiger Form am Boden des Reaktors, während das Gas seitwärts am Ende des Reaktors abgezogen wird, einen Wärmetauscher durchströmt und dann mittels eines Venturiwäschers auf 30 °C abgekühlt wird. Hierbei wird gleichzeitig der Ruß ausgewaschen, so daß im Abgas kein Kohlenstoff nachgewiesen werden kann. Aus dem ablaufenden Wasser wird eine Probe genommen und der Gehalt an festem Kohlenstoff bestimmt.

Aus der Wassermenge und dem C-Gehalt läßt sich ein Vergasungsgrad von 70 % errechnen, d. h. der Vergasungsgrad des Kokses ist für ein technisch durchführbares Verfahren zu gering.

Die Ausbeute an Synthese- oder Reduktionsgas ($CO + H_2$) beträgt 2,6 Nm$^3$/kg Koks.

**Beispiel 1**

Der Aufbau der Anlage und der Reaktionsbedingungen entsprechen denjenigen im Vergleichsbeispiel A bis auf die Ausnahme, daß die Verweilzeit in der Pyrolysezone von 13 auf 2 msec reduziert wird. Die Zunahme der Gasmenge beträgt jetzt nur 45 kg, d. h. das 1,5fache der Flüchtigen der Kohle.

Die Acetylenausbeute beträgt jetzt 32 % und der spez. Energiebedarf beträgt 11,3 kWh/kg $C_2H_2$. Die gleiche Koksmenge, wie im Vergleichsbeispiel A, wird jetzt dem 2. Reaktor unter gleichen Betriebsbedingungen zugeführt. Aus der C-Bestimmung und der Wassermenge ergibt sich jetzt ein Vergasungsgrad des Kokses von 97 %, wie er bei technischen Verfahren zur Kohlevergasung üblich ist. Die Ausbeute an Synthese- oder Reduktionsgas beträgt 3,6 Nm$^3$/kg Koks. Die Gasaufarbeitung in beiden Stufen erfolgt in bekannter Weise.

**Beispiel 2**

In einem einstufigen Lichtbogenofen von 360 kW, der mit einem Gemisch von 80 % $H_2$, 19 % CO, das aus der Vergasungsstufe gewonnen wird und 1 % $CH_4$ als Plasmagas bei einer Energiedichte von 2,8 kWh/Nm$^3$ und einem Druck von 0,5 bar betrieben wird, wird feingemahlene Kohle mit einem Gehalt an Flüchtigen von 25 % in einer Menge von 120 kg/h, wasser- und aschefrei gerechnet, mit einem Gas gleicher Zusammensetzung wie das Plasmagas, eingedüst und bei 2 200 °C pyrolysiert.

Nach einer Verweilzeit von 2 msec wird das Gas mit einem Wasserquench zunächst auf 600 °C und dann mit einem Abhitzekessel auf 200 °C abgekühlt und Koks und Gas wie im Vergleichsbeispiel A abgetrennt. Die Gasausbeute beträgt 42 %, d. h. das 1,68fache der flüchtigen Bestandteile der Kohle. Die Acetylenausbeute beträgt 27 % bei einem spez. Energiebedarf von 11,1 kWh/kg $C_2H_2$.

Die gleichen Koksmengen wie im Vergleichsbeispiel A werden jetzt dem 2. Lichtbogenreaktor unter den gleichen Betriebsbedingungen zugeführt. Aus der C-Bestimmung und der Abwassermenge ergibt sich ein Vergasungsgrad von 92 %; die Ausbeute an Synthesegas beträgt 3,4 Nm$^3$/kg. Die Gasaufarbeitung in beiden Stufen erfolgt in bekannter Weise.

### Beispiel 3

Koks, wie er nach Beispiel 1 gewonnen wurde, wird in einer Menge von 60 kg/h mittels eines Gasgemisches gemäß Vergleichsbeispiel A in den Reaktor, ebenfalls Vergleichsbeispiel A, eingeführt. In diesen Reaktor strömt, ebenfalls wie im Vergleichsbeispiel A, ein Plasmastrahl, der in einem Lichtbogenreaktor mit einer Leistung von 300 kW aus einem Gasgemisch, bestehend aus 60 Vol.% $H_2$, 25 Vol.% $H_2O$, 10 Vol.% $CO_2$ und 5 Vol.% CO erzeugt wird. Die Energiedichte beträgt 3,2 $kWh/Nm^3$ Gasgemisch. Zusätzlich wird, wie im Vergleichsbeispiel A, Wasserdampf zugeführt, so daß das gesamte Verhältnis O/C 1,2 beträgt. Die Verweilzeit des Gases beträgt 5 sec und die Ausgangstemperatur 1 350 °C. Die übrigen Bedingungen und die Gasaufarbeitung wird wie im Vergleichsbeispiel A durchgeführt. Es wird ein Vergasungsgrad von 95 % erreicht, was einer Ausbeute an Synthese- oder Reduktionsgas von 3,5 $Nm^3/kg$ Koks entspricht.

### Patentansprüche

1. Verfahren zur Herstellung von Acetylen und Synthese- oder Reduktionsgas aus Kohle mittels eines Lichtbogen- oder Plasmaverfahrens, dadurch gekennzeichnet, daß man pulverförmig aufbereitete Kohle in einem ersten Lichtbogenreaktor bei einer Energiedichte von 1 bis 5 $kWh/Nm^3$, einer Verweilzeit von 0,5 bis 10 msec und Temperaturen von mindestens 1 500 °C so pyrolysiert, daß die aus der Kohle gewonnenen, gasförmigen Verbindungen das 1,8fache der sogenannten "Flüchtigen" der Kohle nicht übersteigen, man den nach dem Quenchen zurückbleibenden Koks dann einem zweiten Lichtbogenreaktor zuführt, in dem man den Koks mittels eines Vergasungsmittels und Aufheizen in einem Lichtbogen- oder Plasmaverfahren bei einer Verweilzeit von 1 bis 15 sec und einer Temperatur von mindestens 800 °C zu Synthese- oder Reduktionsgas umsetzt und die in der Pyrolyse- und Vergasungsstufe erhaltenen Gase in bekannter Weise aufarbeitet, indem der Gasstrom aus der Pyrolysestufe gereinigt und aus diesem das Acetylen durch selektive Lösungsmittel gewonnen wird und das Gas aus der Vergasungsstufe gegebenenfalls nach Abkühlung gereinigt wird.

2. Verfahren nach Anspruch 1, dadurch gekennzeichnet, daß man die mittlere Temperatur in der Pyrolysestufe von 1 500 bis 3 000 °C und/oder in der Vergasungsstufe von 800 bis 1 700 °C hält.

3. Verfahren nach Anspruch 1, dadurch gekennzeichnet, daß man das CO/$H_2$-haltige Abgas aus der Pyrolysestufe nach Abtrennung des Acetylens in der Aufarbeitung des Abgases aus der Vergasungsstufe einsetzt.

4. Verfahren nach Anspruch 1, dadurch gekennzeichnet, daß man die $H_2S$-haltigen Gasströme aus beiden Gasreinigungsstufen gemeinsam zu Schwefel aufarbeitet.

5. Verfahren nach Anspruch 1, dadurch gekennzeichnet, daß man den in der Pyrolysestufe bzw. in der ersten Gasreinigungsstufe im Gemisch mit anderen Gasen anfallenden Schwefelkohlenstoff der Vergasungsstufe zuführt.

6. Verfahren nach Anspruch 1, dadurch gekennzeichnet, daß man als Plasmagas in den zweiten Lichtbogenreaktor teilweise oder ganz ein Vergasungsmittel, wie Wasserdampf, Kohlendioxid oder Gemische aus beiden einsetzt.

7. Verfahren nach Anspruch 1 und Anspruch 6, dadurch gekennzeichnet, daß man den Wasserdampf, der als Vergasungsmittel benötigt wird, aus einem der Wärmetauscher aus den Abkühlungsstufen gewinnt.

8. Verfahren nach Anspruch 1 und Anspruch 6, dadurch gekennzeichnet, daß man als Vergasungsmittel in den Gasreinigungsstufen anfallendes Kohlendioxid ganz oder teilweise einsetzt.

### Claims

1. A process for the production of acetylene and synthesis gas or reduction gas from coal by means of an electric arc or plasma process, characterised in that pulverulent dressed coal is pyrolysed in a first arc reactor at an energy density of 1 to 5 $kWh/Nm^3$, a residence time of 0.5 to 10 msec and a temperature of at least 1500° C such that the gaseous compounds recovered from the coal do not exceed 1.8 times the so-called "volatiles" of the coal; the coke remaining after quenching is then passed to a second arc reactor in which the coke is converted to synthesis gas or reduction gas by means of a gasifying agent and heating in an electric arc or plasma process at a residence time of 1 to 15 seconds and a temperature of at least 800° C; and the gases obtained in the pyrolysis and gasification stages are worked up in a known manner in which the gas stream from the pyrolysis stage is purified and the acetylene is recovered from this by a selective solvent and the gas from the gasification stage is purified, optionally after cooling.

2. A process according to claim 1, characterised in that the average temperature is maintained at 1500 to 3000° C in the pyrolysis stage and/or at 800 to 1700° C in the gasification stage.

3. A process according to claim 1, characterised in that the CO-and $H_2$-containing offgas from the pyrolysis stage is introduced into working up of the offgas from the gasification stage after separation of the acetylene from the first-mentioned offgas.

4. A process according to claim 1, characterised in that the $H_2S$-containing gas streams from both gas purification stages are worked up together to form sulphur.

5. A process according to claim 1, characterised in that the carbon disulphide arising in admixture with other gases in the pyrolysis stage and/or the first gas purification stage is passed to the gasification stage.

6. A process according to claim 1, characterised in that a gasifying agent such as steam, carbon dioxide or mixtures thereof is introduced into the second arc reactor as part or all of the plasma gas.

7. A process according to claims 1 or 6, characterised in that the steam which is required as gasifying agent is recovered from one of the heat exchangers from the cooling stages.

8. A process according to claims 1 or 6, characterised in that carbon dioxide arising in the gas purification stages is introduced as part or all of the gasifying agent.

## Revendications

1. Procédé pour préparer à partir de charbon de l'acétylène et du gaz de synthèse ou de réduction en faisant appel à un procédé à arc électrique ou à plasma, caractérisé par le fait que l'on pyrolyse le charbon réduit l'etat de poussier dans un premier réacteur à arc electrique, avec une densité énergétique de 1 à 5 kWh par mètre cube normal, un temps de séjour d'une demie à 10 microsecondes et à des températures de 1 500° C au moins, de manière que les composés gazeux obtenus à partir du charbon ne dépassent pas 1,8 fois la fraction dite "volatile" du charbon, que l'on délivre ensuite le coke restant après l'injection à un second réacteur à arc électrique dans lequel on fait réagir le coke à l'aide d'un agent de gazéification et d'un chauffage dans un procédé à arc électrique ou à plasma avec un temps de séjour d'une à 15 secondes et une température d'au moins 800° C pour obtenir un gaz de synthèse ou de réduction et que l'on traite d'une manière connue en soi les gaz obtenus dans l'étape de pyrolyse et de gazéification, en purifiant le courant gazeux provenant de l'étape de pyrolyse et en obtenant à partir dudit courant gazeux l'acétylène à l'aide de solvants sélectifs et en purifiant, éventuellement après refroidissement, le gaz provenant de l'étape de gazéification.

2. Procédé selon la revendication 1, caractérisé par le fait que la température moyenne dans l'étape de pyrolyse est maintenue a une valeur de 1 500 a 3 000° C et/ou qu'elle est maintenue à une valeur de 800 à 1 700° C dans l'étape de gazéification.

3. Procédé selon la revendication 1, caractérisé par le fait qu'après avoir séparé l'acétylène on utilise, lors du traitement du gaz d'échappement provenant de l'étape de gazéification, le gaz d'échappement renfermant un mélange d'oxyde de carbone et d'hydrogène provenant de l'étape de pyrolyse.

4. Procédé selon la revendication 1, caractérisé par le fait qu'on traite en commun, pour obtenir le soufre, les courants gazeux renfermant de l'hydrogène sulfuré qui proviennent des deux étapes de purification du gaz.

5. Procédé selon la revendication 1, caractérisé par le fait que l'on délivre à l'étape de gazéification l'hydrogène sulfuré se formant en mélange avec d'autres gaz dans l'étape de pyrolyse ou dans la première étape de purification de gaz.

6. Procédé selon la revendication 1, caractérisé par le fait que l'on utilise comme gaz de plasma dans le second reacteur à arc électrique, en partie ou partiellement, un agent de gazéification comme la vapeur d'eau, le dioxyde de carbone ou des mélanges des deux.

7. Procédé selon la revendication 1 et la revendication 6, caractérisé par le fait que l'on obtient la vapeur d'eau nécessaire comme agent de gazéification à partir de l'un des échangeurs de chaleur des étapes de refroidissement.

8. Procédé selon la revendication 1 et la revendication 6, caractérisé par le fait qu'on utilise totalement ou partiellement, comme agent de gazéification, la dioxyde de carbone se formant dans les étapes de purification de gaz.

0 133 982

K

1

2

3 → A

4 5

6 → S

12

7 → Sy/H₂

8 9 10 11

S

R